# EUROPEAN PATENT APPLICATION

(11) **EP 0 937 696 A1**
(43) Date of publication of application: **25.08.1999**
(21) Application number: 99300987.7
(22) Date of filing: 10.02.1999
(51) Int. Cl.: C07B 61/00, C07D 211/94

(54) **Safety-catch linkers**

(30) Priority: 11.02.1998 US 22108
(71) Applicant: Irori, La Jolla, CA 92037 (US)
(72) Inventor: Xiao, Yi-Xiao, San Diego, California 92130 (US); Czarnik, Anthony W., San Diego, California 92127 (US)
(74) Representative: Goldin, Douglas Michael

(57) **Abstract**

Safety-catch linkers and methods for use of safety-catch linkers in solid phase and combinatorial solid phase synthesis are provided. Safety-catch linkers covalently bonded to solid supports are also provided. The safety-catch linkers are stable to a wide variety of synthetic protocols so that substrates, such as amines, thiols and alcohols, are cleaved from the linkers under mild conditions. The safety-catch linkers are useful in solid phase synthesis and modification of natural products and other complex organic substrates.

## Description

### FIELD OF THE INVENTION

The present invention relates to safety-catch linkers useful in solid phase and solid phase combinatorial chemistry for binding a substrate to a resin or other solid support during synthesis. In particular, safety-catch linkers that are activated for cleavage and subsequently cleaved from the substrate under mild conditions are provided.

### BACKGROUND OF THE INVENTION

Solid phase and combinatorial chemistry are very important in the production and screening of collections or "libraries" of compounds. These libraries are of increasing importance in medicinal chemistry and the discovery of new therapeutic agents. See, generally, Backes *et al.* Current Opinion in Chem. Biol. **1997**, 1, 86-93. Although these synthetic methods were initially developed for the generation of peptides and oligonucleotides, focus has recently shifted to the area of small molecule synthesis, due to the improved pharmacokinetics of such compounds and the greater potential of small molecules as therapeutic agents.

A key component of any solid phase or solid phase combinatorial chemical synthetic strategy is the linker used to bind the substrate to a resin, such as a polystyrene resin, or other solid support. Selection of the proper linker can be key to the success of the synthetic strategy since the linker must be easily attached to both the substrate and the resin or other solid support, stable to the reaction conditions used in the chemical modification of the substrate and easily removed from the modified substrate to provide the desired library of compounds.

Initially linkers initially were based on protecting groups used in solution phase synthesis (see, e.g., Leznoff *et al.* Can. J. Chem. **1972**, 50, 2892-2893; Frechet *et al.* Can. J. Chem. **1976**, 54, 926-934; Chan *et al.* J. Chem. Soc., Chem. Commun. **1995**, 1475-1476; Krchnak *et al.* Mol. Divers. **1995**, 1, 149-164; Burgess *et al.* J. Org. Chem. **1997**, 62, 5165-5168). These linkers, however, suffer from the limitations placed on subsequent chemistry because of their instability in certain reaction conditions. Additionally, the released elaborated substrates generally contain a polar functional group that serves as the point of attachment of the substrate to the linker. Such polar functionality often has adverse affects on the pharmacokinetics of the resulting compounds.

To avoid this problem, "traceless" linkers were developed (see, e.g., Plunkett *et al.* J. Org. Chem. **1995**, 60, 6006-6007; Han *et al.* Tetrahedron Lett. **1996**, 37, 2703-2706; Lorsbach *et al.* J. Org. Chem. **1996**, 61, 8716-8717; Newlander *et al.* J. Org. Chem. **1997**, 62, 6726-6732). These linkers have the feature such that, upon removal of the elaborated substrate from the linker, no trace of the point of attachment of the linker to the substrate is apparent.

Other known linker strategies include the use of support-bound chiral auxiliaries (see, e.g., Worster *et al.* Angew. Chem. Int. Ed. Eng. **1979**, 18, 221-222; Allin *et al.* Tetrahedron Lett. **1996**, 37, 8023-8026), photolabile linkers (see, e.g., Lloyd-Williams *et al.* Tetrahedron **1993**, 49, 11065-11133; Brown *et al.* Mol. Divers. **1995**, 1, 4-12) and linkers devised to assist in deconvolution of combinatorial libraries (see, e.g., Salmon *et al.* Proc. Natl. Acad. Sci. U.S.A. **1993**, 90, 11708-11712; Cardno *et al.* Tetrahedron Lett. **1996**, 37, 135-138). Strategies using these linkers suffer from the lack of chemical stability required for performing a wide range of chemical reactions. Therefore, the usefulness of these linkers in building small molecule libraries is limited.

Another linker strategy employed in combinatorial synthesis is the use of diversification linkers (see, e.g., Kaldor *et al.* Tetrahedron Lett. **1996**, 37, 7193-7196; DeGrado *et al.* J. Org. Chem. **1980**, 45, 1295-1300). These linkers have the common characteristic that, in the cleavage step, diverse substituents are incorporated into the substrate. The conditions required in the cleavage step are, however, incompatible with a variety of substrates and functionality, and, therefore, limit the usefulness of this strategy.

Another type of linker strategy involves a linker which allows for release of the substrate from the resin or other solid support by cyclization onto the support attachment site (see, e.g., Bhargava *et al. J.* Am. Chem. Soc. **1983**, 105, 3247-3251; Van Maarseveen *et al.* Tetrahedron Lett. **1996**, 37, 8249-8252; Entwistle *et al.* Tetrahedron Lett. **1979**, 555-558; Maeji *et al.* J. Immunol. Meth. **1990**, 134, 23-33; Bray *et al.* J. Org. Chem. **1991**, 56, 6659-6666). In this strategy, the linker actually becomes part of the released modified substrate. Therefore, this method is limited not only to the generation of compounds that contain rings (due to the cyclization), but also to compounds which contain groups suitable as linkers. The method also suffers from the problems of competing intersite reactions, substrate dimerization and resin cross-linking.

A solution to some the problems with these linkers has been the development of "safety-catch" linkers (see, e.g., Backes *et al.* J. Am. Chem. Soc. **1996**, 118, 3055-3056; Gayo *et al.* Tetrahedron Lett. **1997**, 38, 211-214; Morphy *et al.* Tetrahedron Lett. **1996**, 37, 3209-3212, Patek *et al.* Tetrahedron Lett. **1991**, 32, 3891-3894; and Routledge *et al.* Tetrahedron Lett. **1997**, 38, 1227-1230), which afford greater chemical stability than other linkers. They are cleaved by an activation-cleavage sequence in which the linker, with the modified substrate and the resin or other solid support attached, is first activated through chemical modification. Subsequent cleavage of the modified substrate from the resin or other solid support may then be accomplished under relatively mild conditions.

There, however, are drawbacks to and limitations on the use of these safety-catch linkers. Because activation requires relatively harsh conditions, such as strong acids and bases, strong oxidants and strong alkylating agents, their use is limited. Many substrates, such as natural products or other complex organic molecules, are not stable to such conditions. Additionally, these safety-catch linkers are a form of diversification linkers in that the reagents used in the mild cleavage step are incorporated in the released modified substrate. While this does provide the opportunity for creating further diversity, it also limits the types of compounds that can be constructed.

Thus, there is a need for linkers that overcome the limitations of those heretofore available. Therefore, it is an object herein to provide safety-catch linkers that are more versatile than those presently available. Specifically, it is an object herein to provide safety-catch linkers that are activated toward cleavage and cleaved form the modified substrate under mild conditions. It is a further object herein to provide safety-catch linkers that are cleavable from the modified substrate under mild conditions without incorporation of the cleavage reagent. It is also an object herein to provide methods for linking substrates to resins and other solid supports using the safety-catch linkers provided herein and subsequently for cleaving the modified substrate from the safety-catch linker.

### SUMMARY OF THE INVENTION

Safety-catch linkers and methods of using the safety-catch linkers are provided. Safety-catch linkers covalently bonded to solid supports are also provided. The safety-catch linkers are carboxylic acids and derivatives, such as, but not limited to, esters, thioesters, thiaesters, dithioesters, imino ethers and imino thioethers, and are useful for attaching substrates, such as, but not limited to, alcohols, amines and thiols, to resins or other solid supports. After modification, the substrates are removed from the linker by activating the linker followed by cleavage. Activation is achieved under a variety of conditions, including, but not limited to, mild reduction. Cleavage is accomplished by treatment under mildly basic conditions. The conditions used for the activation/cleavage sequence are sufficiently mild so that the safety-catch linkers provided herein can be used in solid phase synthesis, combinatorial solid phase synthesis and modification of a wide variety of compounds, including natural products, such as TAXOL, and other complex organic compounds.

The safety-catch linkers provided herein have as a key element X², which is part of a moiety of formula: 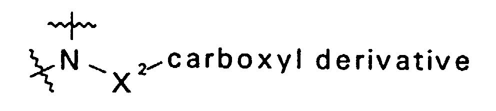 where X² is a group that is conformationally restricted so that the nitrogen atom and the carboxyl derivative at either end of X² are held in proximity to one another such that cyclization of the nitrogen atom onto the carboxyl derivative is facilitated. Such groups contain, for example, 1,2-arylene, 1,2-heteroarylene, 1,1- or 1,2-cycloalkylene, 1,1- or 1,2-heterocyclylene, (*cis*)-alkenylene or gem-dialkyl moieties. Such groups are preferred since substrates are more readily cleaved from safety-catch linkers that possess these groups.

In one embodiment, the safety-catch linkers provided herein have formula I: 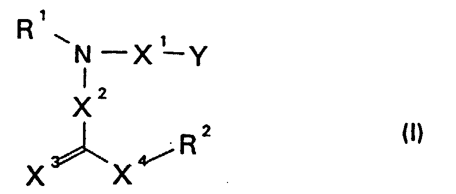 where R¹ is hydrogen or an amine protecting group and R² is hydrogen or a carboxyl protecting group (see, Greene, "Protective Groups in Organic Synthesis" (John Wiley & Sons, Inc., New York, NY)); Y is selected from COOH, NH₂, OH and SH; X¹ and X² are each independently alkylene, alkenylene, alkynylene, arylene, heteroarylene, aralkylene, heteroaralkylene, cycloalkylene, heterocyclylene, alkylenearalkylene, alkyleneheteroaralkylene, alkylenecycloalkylene, alkyleneheterocyclylene, alkylenecycloalkylenealkylene or alkyleneheterocyclylenealkylene, and are unsubstituted or substituted with one or more substituents designated Z, which, as defined herein, is halogen, hydroxy, nitrile, nitro, formyl, mercapto, carboxy, alkyl, haloalkyl, polyhaloalkyl, aminoalkyl, diaminoalkyl, alkenyl containing 1 to 2 double bonds, alkynyl containing 1 to 2 triple bonds, cycloalkyl, cycloalkylalkyl, aryl, heteroaryl, arylalkyl, heteroarylalkyl, alkylidene, arylalkylidene, alkylcarbonyl, arylcarbonyl, heteroarylcarbonyl, alkoxycarbonyl, alkoxycarbonylalkyl, aryloxycarbonyl, aryloxycarbonylalkyl, aminocarbonyl, alkylaminocarbonyl, dialkylaminocarbonyl, arylaminocarbonyl, diarylaminocarbonyl, arylalkylaminocarbonyl, alkoxy, aryloxy, perfluoroalkoxy, alkenyloxy, alkynyloxy, arylalkoxy, amino, aminoalkyl, alkylaminoalkyl, dialkylaminoalkyl, arylaminoalkyl, diarylaminoalkyl, alkylamino, dialkylamino, arylamino, diarylamino, alkylarylamino, alkylcarbonylamino, alkoxycarbonylamino, arylcarbonylamino, aryloxycarbonylamino, azido, alkylthio, arylthio, perfluoroalkylthio, thiocyano, isothiocyano, alkylsulfinyl, alkylsufonyl, arylsulfinyl, arylsulfonyl, aminosulfonyl, alkylaminosulfonyl, dialkylaminosulfonyl, arylaminosylfonyl or diarylaminosulfonyl, or two Z substituents may together form alkylene, alkenylene or alkynylene; X³ is selected from oxy, thio and NR²⁰, where R²⁰ is hydrogen, alkyl, aryl, heteroaryl, cycloalkyl or heterocyclyl; and X⁴ is oxy or thio.

In preferred embodiments, R¹ is selected from hydrogen and amine protecting groups, such as, but not limited to, alkoxycarbonyl, aryloxycarbonyl, arylalkoxycarbonyl, heteroaryloxycarbonyl, cycloalkoxycarbonyl, heterocyclyloxycarbonyl, heteroarylalkoxycarbonyl, cycloalkylalkoxycarbonyl, heterocyclylalkoxycarbonyl, arylalkyl, diarylalkyl, triarylalkyl, alkylcarbonyl, arylcarbonyl, arylalkylcarbonyl, heteroarylcarbonyl, heteroarylalkylcarbonyl, R³R⁴NOC(O)- and R³S(O)ₙ-; R³ and R⁴ are each independently selected from alkyl, alkenyl, alkynyl, aryl, arylalkyl, heteroaryl and heteroarylalkyl, or together form alkylene or alkenylene; n is 0-2; R² is hydrogen or a carboxyl protecting group, such as, but not limited to, alkyl, alkenyl, alkynyl, aryl, heteroaryl, aralkyl, heteroaralkyl, cycloalkyl, heterocyclyl, trialkylsilyl, aryldialkylsilyl, diarylalkylsilyl and triarylsilyl; and R¹ and R² are unsubstituted or substituted with one or more Z substituents.

In more preferred embodiments, X² is a group that is conformationally restricted so that the nitrogen atom and the carboxyl group at either end of X² are held in proximity to one another. Such groups contain, for example, 1,2-arylene, 1,2-heteroarylene, 1,1- or 1,2-cycloalkylene, 1,1- or 1,2-heterocyclylene, (*cis*)-alkenylene or gem-dialkyl moieties. Such groups are preferred since substrates are more readily cleaved from safety-catch linkers that possess these groups.

In preferred embodiments described in detail herein, X² is alkylene, alkenylene, alkynylene, arylene, heteroarylene, aralkylene, heteroaralkylene, cycloalkylene, heterocyclylene, alkylenearalkylene, alkyleneheteroaralkylene, alkylenecycloalkylene, alkyleneheterocyclylene, alkylenecycloalkylenealkylene or alkyleneheterocyclylenealkylene, is unsubstituted or substituted with one or more Z substituents, and is conformationally restricted so that the nitrogen atom and the carboxyl group at either end of X² are held in proximity to one another.

Methods are provided for use of the safety-catch linkers provided herein. In particular, methods for use of the safety-catch linkers provided herein in solid phase and solid phase combinatorial synthesis are provided. The methods involve linking of a substrate to a solid support, such as a resin, using a safety-catch linker provided herein and, following modification of the substrate, cleaving the modified substrate from the safety-catch linker by a cyclization protocol. In certain embodiments, cleavage of the modified substrate from the safety-catch linker involves a two-step process of (a) activation of the linker through removal of R¹; and (b) cleavage through cyclization induced by treatment with mild base.

In preferred embodiments of the methods, suitable substrates include, but are not limited to, alcohols, amines and thiols. Mild bases include, but are not limited to, amines, or alkali or alkaline earth metal phosphates, hydrogen phosphates, dihydrogen phosphates, hydroxides, carbonates or hydrogen carbonates.

### DETAILED DESCRIPTION OF PREFERRED EMBODIMENTS

### Definitions

Unless defined otherwise, all technical and scientific terms used herein have the same meaning as is commonly understood by one of skill in the art to which this invention belongs. All patents and publications referred to herein are incorporated by reference.

As used herein, a linker is a compound that is used to bind a substrate molecule to a solid support, such as a resin.

As used herein, a safety-catch linker is a linker that requires activation through chemical modification prior to cleavage of the substrate from the resin-bound linker. Safety-catch linkers are stable to most reagents and solvents, thereby allowing for their use in a wide variety of synthetic strategies.

As used herein, an amine protecting group is any amine derivative, covalent or ionic or otherwise, which prevents the amine from participating in reactions being selectively conducted at other sites in the molecule. Examples of amine protecting groups include, but are not limited to, carbamates, amides, ureas, imides, N-alkyl derivatives, metal chelates, amino acetal derivatives, imines, enamines, oxides, nitros, nitrosos, N-phosphorous derivatives, aminosilanes, sulfenyls, sulfinyls and sulfonyls.

As used herein, a carboxyl protecting group is any carboxyl derivative, covalent or ionic or otherwise, which prevents the carboxyl group from participating in reactions being selectively conducted at other sites in the molecule. Examples of carboxyl protecting groups include, but are not limited to, esters, including but not limited to, alkyl, aryl, aralkyl, silyl, thio, heteroaryl and stannyl esters, amides, hydrazides and other such groups known to those of skill in the art.

As used herein, carboxylic acid derivatives, include any such groups known to those of skill in the art, such as, but not limited to, esters, thioesters, thiaesters, dithioesters, imino ethers and imino thioethers.

As used herein, mild conditions refer to conditions which do not affect or alter most functional groups, such as, but not limited to, carboxylic acids, amides and esters; alkyl halides; ethers; alkyl amines; ketones; alkenes; alkynes; nitro, azido, sulfinyl and sulfonyl groups; alcohols; amines; thiols; and aldehydes.

As used herein, a mild base is any compound where the pKa of the conjugate acid is less that 20, preferably less than 10, more preferably less than 5. As used herein, "mildly basic conditions" involve treatment with a mild base.

As used herein, "mild reduction" means reducing conditions which will not reduce most organic functional groups. Mild reductions generally will not reduce amides, esters, carboxylic acids, alkyl halides, ethers, alkyl amines or ketones. Exemplary mild reductions include treatment with sodium dithionite or homogeneous hydrogenation over a transition metal catalyst such as palladium.

As used herein, "facilitation" of covalent bond formation or cyclization refers to reaction conditions or structural features wherein the rate of covalent bond formation or cyclization is higher than the rate in the absence of the reaction conditions or structural features. A cyclization or covalent bond formation is "facilitated" by a reaction condition or structural feature if the rate of the cyclization or covalent bond formation is higher than the rate in the absence of the reaction condition or structural feature.

As used herein, a microreactor refers to combinations of matrices with memories with associated, such as linked or proximate, biological particles or molecules. It is produced, for example, when the molecule is linked thereto or synthesized thereon. It is then used in subsequent protocols, such as immunoassays and scintillation proximity assays.

As used herein, alkyl, alkenyl and alkynyl carbon chains, if not specified contain from 1 to 20 carbons, preferably 1 to 16 carbons, and are straight or branched. Alkenyl carbon chains of from 1 to 20 carbons preferably contain 1 to 8 double bonds, and the alkenyl carbon chains of 1 to 16 carbons preferably contain 1 to 5 double bonds. Alkynyl carbon chains of from 1 to 20 carbons preferably contain 1 to 8 triple bonds, and the alkynyl carbon chains of 1 to 16 carbons preferably contain 1 to 5 triple bonds. The alkyl, alkenyl and alkynyl groups may be optionally substituted, with one or more groups, preferably alkyl group substituents that may be the same or different. As used herein, lower alkyl, lower alkenyl, and lower alkynyl refer to carbon chains having less than about 6 carbons.

As used herein, an alkyl group substituent includes halo, haloalkyl, preferably halo lower alkyl, aryl, hydroxy, alkoxy, aryloxy, alkyloxy, alkylthio, arylthio, aralkyloxy, aralkylthio, carboxy alkoxycarbonyl, oxo and cycloalkyl.

As used herein, "aryl" refers to cyclic groups containing from 3 to 19 carbon atoms. Aryl groups include, but are not limited to groups, such as phenyl, substituted phenyl, naphthyl, substituted naphthyl, in which the substituent is lower alkyl, halogen, or lower alkoxy.

As used herein, an "aryl group substituent" includes alkyl, cycloalkyl, cycloalkylalkyl, aryl, heteroaryl optionally substituted with 1 or more, preferably 1 to 3, substituents selected from halo, halo alkyl and alkyl, arylalkyl, heteroarylalkyl, alkenyl containing 1 to 2 double bonds, alkynyl containing 1 to 2 triple bonds, halo, hydroxy, haloalkyl and polyhaloalkyl, preferably halo lower alkyl, especially trifluoromethyl, formyl, alkylcarbonyl, arylcarbonyl that is optionally substituted with 1 or more, preferably 1 to 3, substituents selected from halo, halo alkyl and alkyl, heteroarylcarbonyl, carboxy, alkoxycarbonyl, aryloxycarbonyl, aminocarbonyl, alkylaminocarbonyl, dialkylaminocarbonyl, arylaminocarbonyl, diarylaminocarbonyl, arylalkylaminocarbonyl, alkoxy, aryloxy, perfluoroalkoxy, alkenyloxy, alkynyloxy, arylalkoxy, aminoalkyl, alkylaminoalkyl, dialkylaminoalkyl, arylaminoalkyl, amino, alkylamino, dialkylamino, arylamino, alkylarylamino, alkylcarbonylamino, arylcarbonylamino, azido, nitro, mercapto, alkylthio, arylthio, perfluoroalkylthio, thiocyano, isothiocyano, alkylsulfinyl, alkylsufonyl, arylsulfinyl, arylsulfonyl, aminosulfonyl, alkylaminosulfonyl, dialkylaminosulfonyl and arylaminosulfonyl.

As used herein, "arylalkyl" refers to an alkyl group which substituted with one or more aryl groups. Examples of arylalkyl groups include benzyl, 9-fluorenylmethyl, naphthylmethyl, diphenylmethyl and triphenylmethyl.

As used herein, "cycloalkyl" refers to a saturated mono- or multicyclic ring system, preferably of 3 to 10 carbon atoms, more preferably 3 to 6 carbon atoms; cycloalkenyl and cycloalkynyl refer to mono- or multicyclic ring systems that respectively include at least one double bond and at least one triple bond. Cycloalkenyl and cycloalkynyl groups may preferably contain 3 to 10 carbon atoms, with cycloalkenyl groups more preferably containing 4 to 7 carbon atoms and cycloalkynyl groups more preferably containing 8 to 10 carbon atoms. The ring systems of the cycloalkyl, cycloalkenyl and cycloalkynyl groups may be composed of one ring or two or more rings which may be joined together in a fused, bridged or spiro-connected fashion, and may be optionally substituted with one or more alkyl group substituents.

As used herein, "heteroaryl" refers to a monocyclic or multicyclic ring system, preferably of about 5 to about 15 members where one or more, more preferably 1 to 3 of the atoms in the ring system is a heteroatom, i.e., an element other than carbon, for example, nitrogen, oxygen and sulfur atoms. The heteroaryl may be optionally substituted with one or more, preferably 1 to 3, aryl group substituents. Exemplary heteroaryl groups include, for example, furyl, thienyl, pyridyl, pyrrolyl, N-methylpyrrolyl, quinolinyl and isoquinolinyl, with pyridyl and quinolinyl being preferred.

As used herein, "heterocyclic" refers to a monocyclic or multicyclic ring system, preferably of 3 to 10 members, more preferably 4 to 7 members, even more preferably 5 to 6 members, where one or more, preferably 1 to 3 of the atoms in the ring system is a heteroatom, i.e., an element other than carbon, for example, nitrogen, oxygen and sulfur atoms. The heterocycle may be optionally substituted with one or more, preferably 1 to 3 aryl group substituents. Preferred substituents of the heterocyclic group include hydroxy, alkoxy containing 1 to 4 carbon atoms, halo lower alkyl, including trihalomethyl, such as trifluoromethyl, and halogen. As used herein, the term heterocycle may include reference to heteroaryl. Exemplary heterocycles include, for example, pyrrolidinyl, piperidinyl, alkylpiperidinyl, morpholinyl, oxadiazolyl or triazolyl.

As used herein, the nomenclature alkyl, alkoxy, carbonyl, etc. are used as is generally understood by those of skill in this art. For example, as used herein alkyl refers to saturated carbon chains that contain one or more carbons; the chains may be straight or branched or include cyclic portions or be cyclic. As used herein, alicyclic refers to aryl groups that are cyclic.

As used herein, "halogen" or "halide" refers to F, Cl, Br or I.

As used herein, pseudohalides are compounds that behave substantially similar to halides. Such compounds can be used in the same manner and treated in the same manner as halides (X⁻, in which X is a halogen, such as Cl or Br). Pseudohalides include, but are not limited to cyanide, cyanate, thiocyanate, selenocyanate, trifluoromethyl and azide.

As used herein, "haloalkyl" refers to a lower alkyl radical in which one or more of the hydrogen atoms are replaced by halogen including, but not limited to, chloromethyl, trifluoromethyl, 1-chloro-2-fluoroethyl and the like.

As used herein, "haloalkoxy" refers to RO- in which R is a haloalkyl group.

As used herein, "sulfinyl" refers to -S(O)-. As used herein, "sulfonyl" refers to -S(O)₂-.

As used herein, "aminocarbonyl" refers to -C(O)NH₂.

As used herein, "alkylaminocarbonyl" refers to -C(O)NHR in which R is hydrogen or alkyl, preferably lower alkyl. As used herein "dialkylaminocarbonyl" as used herein refers to -C(O)NR'R in which R' and R are independently selected from hydrogen or alkyl, preferably lower alkyl; "carboxamide" refers to groups of formula -NR'COR. As used herein, "diarylaminocarbonyl" refers to -C(O)NRR' in which R and R' are independently selected from aryl, preferably lower aryl, more preferably phenyl. As used herein, "arylalkylaminocarbonyl" refers to -C(O)NRR' in which one of R and R' is aryl, preferably lower aryl, more preferably phenyl, and the other of R and R' is alkyl, preferably lower alkyl. As used herein, "arylaminocarbonyl" refers to -C(O)NHR in which R is aryl, preferably lower aryl, more preferably phenyl.

As used herein, "alkoxycarbonyl" refers to -C(O)OR in which R is alkyl, preferably lower alkyl.

As used herein, "aryloxycarbonyl" refers to -C(O)OR in which R is aryl, preferably lower aryl, more preferably phenyl.

As used herein, "alkoxy" and "thioalkoxy" refer to RO- and RS-, in which R is alkyl, preferably lower alkyl.

As used herein, "aryloxy" and "thioaryloxy" refer to RO- and RS-, in which R is aryl, preferably lower aryl, more preferably phenyl.

As used herein, "alkylene" refers to a straight, branched or cyclic, preferably straight or branched, bivalent aliphatic hydrocarbon group, preferably having from 1 to about 20 carbon atoms, more preferably 1 to 12 carbons, even more preferably lower alkylene. The alkylene group is optionally substituted with one or more "alkyl group substituents." There may be optionally inserted along the alkylene group one or more oxygen, sulphur or substituted or unsubstituted nitrogen atoms, where the nitrogen substituent is alkyl as previously described. Exemplary alkylene groups include methylene (-CH₂-), ethylene (-CH₂CH₂-), propylene (-(CH₂)₃-), cyclohexylene (-C₆H₁₀-), methylenedioxy (-O-CH₂-O-) and ethylenedioxy (-O-(CH₂)₂-O-). The term "lower alkylene" refers to alkylene groups having 1 to 6 carbons. Preferred alkylene groups are lower alkylene, with alkylene of 1 to 3 carbon atoms being particularly preferred.

As used herein, "alkenylene" refers to a straight, branched or cyclic, preferably straight or branched, bivalent aliphatic hydrocarbon group, preferably having from 1 to about 20 carbon atoms and at least one double bond, more preferably 1 to 12 carbons, even more preferably lower alkenylene. The alkenylene group is optionally substituted with one or more "alkyl group substituents." There may be optionally inserted along the alkenylene group one or more oxygen, sulphur or substituted or unsubstituted nitrogen atoms, where the nitrogen substituent is alkyl as previously described. Exemplary alkenylene groups include -CH = CH-CH = CH- and -CH = CH-CH₂-. The term "lower alkenylene" refers to alkenylene groups having 2 to 6 carbons. Preferred alkenylene groups are lower alkenylene, with alkenylene of 3 to 4 carbon atoms being particularly preferred.

As used herein, "alkynylene" refers to a straight, branched or cyclic, preferably straight or branched, bivalent aliphatic hydrocarbon group, preferably having from 1 to about 20 carbon atoms and at least one triple bond, more preferably 1 to 12 carbons, even more preferably lower alkynylene. The alkynylene group is optionally substituted with one or more "alkyl group substituents." There may be optionally inserted along the alkynylene group one or more oxygen, sulphur or substituted or unsubstituted nitrogen atoms, where the nitrogen substituent is alkyl as previously described. Exemplary alkynylene groups include -C≡C-C≡C-, -C≡C- and -C≡C-CH₂-. The term "lower alkynylene" refers to alkynylene groups having 2 to 6 carbons. Preferred alkynylene groups are lower alkynylene, with alkynylene of 3 to 4 carbon atoms being particularly preferred.

As used herein, "arylene" refers to a monocyclic or polycyclic, preferably monocyclic, bivalent aromatic group, preferably having from 1 to about 20 carbon atoms and at least one aromatic ring, more preferably 1 to 12 carbons, even more preferably lower arylene. The arylene group is optionally substituted with one or more "alkyl group substituents." There may be optionally inserted around the arylene group one or more oxygen, sulphur or substituted or unsubstituted nitrogen atoms, where the nitrogen substituent is alkyl as previously described. Exemplary arylene groups include 1,2-, 1,3- and 1,4-phenylene. The term "lower arylene" refers to arylene groups having 5 or 6 carbons. Preferred arylene groups are lower arylene.

As used herein, "heteroarylene" refers to a bivalent monocyclic or multicyclic ring system, preferably of about 5 to about 15 members where one or more, more preferably 1 to 3 of the atoms in the ring system is a heteroatom, i.e., an element other than carbon, for example, nitrogen, oxygen and sulfur atoms. The heteroarylene group may be optionally substituted with one or more, preferably 1 to 3, aryl group substituents. Exemplary heteroarylene groups include, for example, 1,4-imidazolylene.

As used herein, "alkylidene" refers to a bivalent group, such as =CR'R", that is attached to one atom of another group, forming a double bond. Exemplary alkylidene groups are methylidene (=CH₂) and ethylidene (=CHCH₃).

As used herein, "arylalkylidene" refers to an alkylidene group in which either R' or R" is an aryl group.

As used herein, "amido" refers to a bivalent group, either -C(O)NH- or -HNC(O)-. "Thioamido" refers to either -C(S)NH- or -HNC(S)-. "Oxyamido" refers to either -OC(O)NH- or -HNC(O)O-. "Thiaamido" refers to either -SC(O)NH- or -HNC(O)S-. "Dithiaamido" refers to either -SC(S)NH- or -HNC(S)S-. "Ureido" refers to -HNCONH-. "Thioureido" refers to -HNCSNH-. "Thioester" refers to -C(O)S- or -SC(O)-. "Thiaester" refers to -C(S)O- or -OC(S)-. "Dithioester" refers to -C(S)S- or -SC(S)-. "Imino ether" refers to -N(=R)O- or -ON( = R)-. "Imino thioether" refers to -N(=R)S- or -SN(=R)-.

As used herein, the term "amino acid" refers to α-amino acids that are racemic, or of either the D- or L-configuration.

As used herein, when any particular group, such as phenyl or pyridyl, is specified, this means that the group is unsubstituted or is substituted. Preferred substituents where not specified are halo, halo lower alkyl, and lower alkyl.

As used herein, the abbreviations for any protective groups, amino acids and other compounds, are, unless indicated otherwise, in accord with their common usage, recognized abbreviations, or the IUPAC-IUB Commission on Biochemical Nomenclature (see, Biochem. **1972**, 11, 1726).

### A. Safety-Catch Linkers

Safety-catch linkers and safety-catch linkers covalently bonded to solid supports are provided. The safety-catch linkers are carboxylic acids and derivatives, such as, but not limited to, esters, thioesters, thiaesters, dithioesters, imino ethers and imino thioethers, and are useful for attaching substrates, such as, but not limited to, alcohols, amines and thiols, to resins or other solid supports. After modification, the substrates are removed from the linker by activating the linker followed by cleavage. Activation is achieved under a variety of conditions, including, but not limited to, mild reduction. Cleavage is accomplished by treatment under mildly basic conditions. The safety-catch linkers provided herein are stable to a wide variety of reaction conditions, yet allow for cleavage of the modified substrate from the resin or other solid support under mild conditions. These linkers, therefore, have wide applicability in the area of solid phase synthesis, combinatorial solid phase synthesis and modification of complex organic substrates, including natural products, such as TAXOL, and other complex organic compounds.

The safety-catch linkers provided herein have as a key element X², which is part of a moiety of formula: 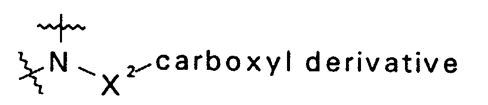 where X² is a group that is conformationally restricted so that the nitrogen atom and the carboxyl derivative at either end of X² are held in proximity to one another such that cyclization of the nitrogen atom onto the carboxyl derivative is facilitated. Such groups contain, for example, 1,2-arylene, 1,2-heteroarylene, 1,1- or 1,2-cycloalkylene, 1,1- or 1,2-heterocyclylene, (cis)-alkenylene or gem-dialkyl moieties. Such groups are preferred since substrates are more readily cleaved from safety-catch linkers that possess these groups.

In one embodiment, the safety-catch linkers provided herein have formula I: 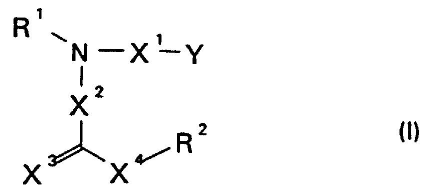 where R¹ is hydrogen or an amine protecting group and R² is hydrogen or a carboxyl protecting group (see, Greene, "Protective Groups in Organic Synthesis" (John Wiley & Sons, Inc., New York, NY)); Y is selected from COOH, NH₂, OH and SH; X¹ and X² are each independently alkylene, alkenylene, alkynylene, arylene, heteroarylene, aralkylene, heteroaralkylene, cycloalkylene, heterocyclylene, alkylenearalkylene, alkyleneheteroaralkylene, alkylenecycloalkylene, alkyleneheterocyclylene, alkylenecycloalkylenealkylene or alkyleneheterocyclylenealkylene, and are unsubstituted or substituted with one or more substituents designated Z, which, as defined herein, is halogen, hydroxy, nitrile, nitro, formyl, mercapto, carboxy, alkyl, haloalkyl, polyhaloalkyl, aminoalkyl, diaminoalkyl, alkenyl containing 1 to 2 double bonds, alkynyl containing 1 to 2 triple bonds, cycloalkyl, cycloalkylalkyl, aryl, heteroaryl, arylalkyl, heteroarylalkyl, alkylidene, arylalkylidene, alkylcarbonyl, arylcarbonyl, heteroarylcarbonyl, alkoxycarbonyl, alkoxycarbonylalkyl, aryloxycarbonyl, aryloxycarbonylalkyl, aminocarbonyl, alkylaminocarbonyl, dialkylaminocarbonyl, arylaminocarbonyl, diarylaminocarbonyl, arylalkylaminocarbonyl, alkoxy, aryloxy, perfluoroalkoxy, alkenyloxy, alkynyloxy, arylalkoxy, amino, aminoalkyl, alkylaminoalkyl, dialkylaminoalkyl, arylaminoalkyl, diarylaminoalkyl, alkylamino, dialkylamino, arylamino, diarylamino, alkylarylamino, alkylcarbonylamino, alkoxycarbonylamino, arylcarbonylamino, aryloxycarbonylamino, azido, alkylthio, arylthio, perfluoroalkylthio, thiocyano, isothiocyano, alkylsulfinyl, alkylsufonyl, arylsulfinyl, arylsulfonyl, aminosulfonyl, alkylaminosulfonyl, dialkylaminosulfonyl, arylaminosylfonyl or diarylaminosulfonyl, or two Z substituents may together form alkylene, alkenylene or alkynylene; X³ is selected from oxy, thio and NR²⁰, where R²⁰ is hydrogen, alkyl, aryl, heteroaryl, cycloalkyl or heterocyclyl; and X⁴ is oxy or thio.

In preferred embodiments, R¹ is selected from hydrogen and amine protecting groups, such as, but not limited to, alkoxycarbonyl, aryloxycarbonyl, arylalkoxycarbonyl, heteroaryloxycarbonyl, cycloalkoxycarbonyl, heterocyclyloxycarbonyl, heteroarylalkoxycarbonyl, cycloalkylalkoxycarbonyl, heterocyclylalkoxycarbonyl, arylalkyl, diarylalkyl, triarylalkyl, alkylcarbonyl, arylcarbonyl, arylalkylcarbonyl, heteroarylcarbonyl, heteroarylalkylcarbonyl, R³R⁴NOC(O)- and R³S(O)ₙ-; R³ and R⁴ are each independently selected from alkyl, alkenyl, alkynyl, aryl, arylalkyl, heteroaryl and heteroarylalkyl, or together form alkylene or alkenylene; n is 0-2; R² is hydrogen or a carboxyl protecting group, such as, but not limited to, alkyl, alkenyl, alkynyl, aryl, heteroaryl, aralkyl, heteroaralkyl, cycloalkyl, heterocyclyl, trialkylsilyl, aryldialkylsilyl, diarylalkylsilyl and triarylsilyl; and R¹ and R² are unsubstituted or substituted with one or more Z substituents.

In more preferred embodiments, X² is a group that is conformationally restricted so that the nitrogen atom and the carboxyl group at either end of X² are held in proximity to one another. Such groups contain, for example, 1,2-arylene, 1,2-heteroarylene, 1,1- or 1,2-cycloalkylene, 1,1- or 1,2-heterocyclylene, (*cis*)-alkenylene or gem-dialkyl moieties. Such groups are preferred since substrates are more readily cleaved from safety-catch linkers that possess these groups.

In preferred embodiments described in detail herein, X² is alkylene, alkenylene, alkynylene, arylene, heteroarylene, aralkylene, heteroaralkylene, cycloalkylene, heterocyclylene, alkylenearalkylene, alkyleneheteroaralkylene, alkylenecycloalkylene, alkyleneheterocyclylene, alkylenecycloalkylenealkylene or alkyleneheterocyclylenealkylene, is unsubstituted or substituted with one or more Z substituents, and is conformationally restricted so that the nitrogen atom and the carboxyl group at either end of X² are held in proximity to one another.

In a preferred embodiment, the safety-catch linkers provided herein are of formula I, where X² is alkylene group that is substituted with two substituents at the same atom of the alkylene chain. X² is preferably 1,1-, 2,2-, or 3,3-(R¹⁰R¹¹)propylene or 1,1-, 2,2-, 3,3-, or 4,4-(R¹⁰R¹¹)butylene, where R¹⁰ and R¹¹ are each independently alkyl, or together form alkylene or alkylidene. R¹⁰ and R¹¹ are more preferably both methyl, or together from pentylene, butylene, propylene or ethylene.

In another preferred embodiment, the safety-catch linkers are of formula I where X² is (*cis*)-alkenylene. X² is more preferably selected from (*cis*)*-*1-butenylene, (*cis*)-2-butenylene, (*cis*)-3-butenylene, (*cis*)*-*1-propenylene and (*cis*)-2-propenylene.

In another preferred embodiment, the safety-catch linkers are of formula I where X² is aralkylene or alkylenearalkylene. X² is more preferably 1-methylene-1,2-phenylene, 1-ethylene-1,2-phenylene or 1,2-di(methylene)-1,2-phenylene, most preferably 1,2-di(methylene)-1,2-phenylene.

In a preferred embodiment, the safety-catch linkers provided herein have formula II: 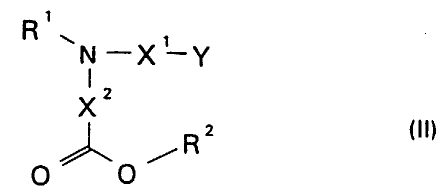 where R¹ is alkoxycarbonyl, aryloxycarbonyl, arylalkoxycarbonyl, heteroaryloxycarbonyl, heterocyclyloxycarbonyl, heteroarylalkoxycarbonyl, heterocyclylalkoxycarbonyl, arylalkyl, diarylalkyl, triarylalkyl, alkylcarbonyl, arylcarbonyl, alkylarylcarbonyl, heteroarylcarbonyl, heteroarylalkylcarbonyl, R³R⁴NOC(O)- or R³S(O)ₙ-; R³ and R⁴ are each independently selected from alkyl, alkenyl, alkynyl, aryl, arylalkyl, heteroaryl and heteroarylalkyl, or together form alkylene or alkenylene; n is 0-2; R² is alkyl, alkenyl, alkynyl, aryl, heteroaryl, aralkyl, heteroaralkyl, cycloalkyl, heterocyclyl, trialkylsilyl, aryldialkylsilyl, diarylalkylsilyl and triarylsilyl; R¹ and R² are unsubstituted or substituted with one or more substituents designated Z, which, as defined herein, is halogen, hydroxy, nitrile, nitro, formyl, mercapto, carboxy, alkyl, haloalkyl, polyhaloalkyl, aminoalkyl, diaminoalkyl, alkenyl containing 1 to 2 double bonds, alkynyl containing 1 to 2 triple bonds, cycloalkyl, cycloalkylalkyl, aryl, heteroaryl, arylalkyl, heteroarylalkyl, alkylidene, arylalkylidene, alkylcarbonyl, arylcarbonyl, heteroarylcarbonyl, alkoxycarbonyl, alkoxycarbonylalkyl, aryloxycarbonyl, aryloxycarbonylalkyl, aminocarbonyl, alkylaminocarbonyl, dialkylaminocarbonyl, arylaminocarbonyl, diarylaminocarbonyl, arylalkylaminocarbonyl, alkoxy, aryloxy, perfluoroalkoxy, alkenyloxy, alkynyloxy, arylalkoxy, amino, aminoalkyl, alkylaminoalkyl, dialkylaminoalkyl, arylaminoalkyl, diarylaminoalkyl, alkylamino, dialkylamino, arylamino, diarylamino, alkylarylamino, alkylcarbonylamino, alkoxycarbonylamino, arylcarbonylamino, aryloxycarbonylamino, azido, alkylthio, arylthio, perfluoroalkylthio, thiocyano, isothiocyano, alkylsulfinyl, alkylsufonyl, arylsulfinyl, arylsulfonyl, aminosulfonyl, alkylaminosulfonyl, dialkylaminosulfonyl, arylaminosylfonyl or diarylaminosulfonyl, or two Z substituents may together from alkylene, alkenylene or alkynylene;
Y is COOH, NH₂, OH or SH;
X¹ and X² are each independently alkylene, alkenylene, alkynylene, arylene, heteroarylene, aralkylene, heteroaralkylene, cycloalkylene, heterocyclylene, alkylenearalkylene, alkyleneheteroaralkylene, alkylenecycloalkylene, alkyleneheterocyclylene, alkylenecycloalkylenealkylene and alkyleneheterocyclylenealkylene, and are unsubstituted or substituted with one or more Z substituents; X¹ is a group which, if a straight chain, is greater than three atoms in length; and X² is a group that is conformationally restricted so that the nitrogen atom and the carboxyl group at either end of X² are held in proximity to one another.

In more preferred embodiments, X² is selected from alkylene, alkenylene, aralkylene, heteroaralkylene, alkylenearalkylene, alkyleneheteroaralkylene, alkylenecycloalkylene, alkyleneheterocyclylene, alkylenecycloalkylenealkylene and alkyleneheterocyclylenealkylene, and is unsubstituted or substituted with one or more Z substituents.

In one particularly preferred embodiment, X² is an alkylene group that is substituted with two substituents at the same atom of the alkylene chain. Thus, the substituents form a gem-dialkyl moiety, or together form alkylene or alkylidene. X² is preferably substituted propylene or butylene and has the formulae 1,1-, 2,2-, or 3,3-(R¹⁰R¹¹)propylene or 1,1-, 2,2-, 3,3-, or 4,4-(R¹⁰R¹¹)butylene, where R¹⁰ and R¹¹ are each independently alkyl, preferably are both methyl, or together form alkylene, preferably pentylene, butylene, propylene or ethylene, or alkylidene.

In another particularly preferred embodiment, X² is an alkenylene group, preferably a (*cis*)-alkenylene group. In a preferred embodiment, X² is a (*cis*)-alkenylene group selected from (*cis*)-1-butenylene, (*cis*)-2-butenylene, (*cis*)-3-butenylene, (*cis*)-1-propenylene and (*cis*)-2-propenylene.

In another particularly preferred embodiment, X² is aralkylene or alkylenearalkylene, preferably 1-methylene-1,2-phenylene, 1-ethylene-1,2-phenylene or 1,2-di(methylene)-1,2-phenylene, more preferably 1,2-di(methylene)-1,2-phenylene.

In more preferred embodiments, R¹ is selected from alkoxycarbonyl, aryloxycarbonyl, arylalkoxycarbonyl, heterocyclyloxycarbonyl, heterocyclylalkoxycarbonyl and R³R⁴NOC(O)-, most preferably R³R⁴NOC(O)-; R³ and R⁴ are each independently selected from alkyl, aryl, aralkyl, heteroaryl and heteroaralkyl, or together form alkylene or alkenylene; R² is alkyl, alkenyl, alkynyl, aryl, heteroaryl, aralkyl, heteroaralkyl, cycloalkyl or heterocyclyl; and X¹ is selected from alkylene, alkenylene, alkynylene, arylene, heteroarylene, aralkylene and heteroaralkylene.

In other more preferred embodiments, Y is COOH. In another more preferred embodiments, Y is NH₂, OH or SH.

In another more preferred embodiment, X¹ is a group which, if a straight chain, is greater than three atoms in length. X¹ thus provides a spacer between the resin or solid support and the point of attachment of the substrate. Such a spacer is advantageous in providing freedom to the attached substrate, and also in reducing the incidence of cross-linking, intersite reactions and substrate dimerization.

Thus, in more preferred embodiments, R¹ is R³R⁴NOC(O)-, where R³ and R⁴ are each independently selected from alkyl, aryl, arylalkyl, heteroaryl and heteroarylalkyl, or together form alkylene or alkenylene; and the safety-catch linkers provided herein have formula III: 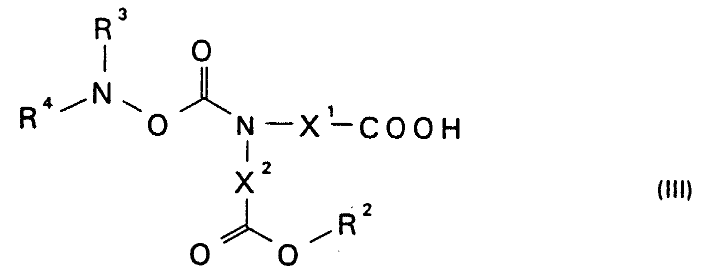 where R³ and R⁴ are each independently alkyl, aryl, heteroaryl, aralkyl and heteroaralkyl, or together form alkylene or alkenylene, and are unsubstituted or substituted with one or more Z substituents; R² is alkyl, alkenyl, alkynyl, aryl, heteroaryl, aralkyl, heteroaralkyl, cycloalkyl or heterocyclyl, and is unsubstituted or substituted with one or more Z substituents;
X¹ is an alkylene, alkenylene, alkynylene, arylene, heteroarylene, aralkylene or heteroaralkylene group, which, if a straight chain, is greater than three atoms in length; and
X² is selected as described above.

In particularly preferred embodiments, the safety-catch linkers are of formula III where R³ and R⁴ are each independently alkyl or aryl, or together form alkylene or alkenylene, and are unsubstituted or substituted with one or more Z substituents; and R² is selected from the group consisting of alkyl, aryl, aralkyl, heteroaryl and heteroaralkyl, and is unsubstituted or substituted with one or more Z substituents. R³ and R⁴ are preferably each independently alkyl or together form alkylene, more preferably each methyl or together form pentylene, most preferably together form pentylene. R² is preferably alkyl or aralkyl, more preferably lower alkyl or aralkyl, such as tert-butyl, benzyl or 9-fluorenylmethyl, most preferably 9-fluorenylmethyl.

In particularly preferred embodiments, X¹ is alkylene, arylene or heteroarylene, preferably alkylene or arylene, more preferably alkylene, most preferably pentylene.

In other embodiments, the safety-catch linkers provided herein are covalently bonded to a resin or other solid support. The resins or other solid supports are any that are suitable for solid phase or combinatorial chemical synthesis, such as, but not limited to, inorganics, natural polymers, and synthetic polymers, including, but not limited to: cellulose, cellulose derivatives, acrylic resins, glass that is derivatized to render it suitable for use a support, silica gels, polystyrene, gelatin, polyvinyl pyrrolidone, co-polymers of vinyl and acrylamide, polystyrene cross-linked with divinylbenzene or the like (see, Merrifield Biochemistry **1964**, 3, 1385-1390), polyacrylamides, latex gels, polystyrene, dextran, rubber, silicon, plastics, nitrocellulose, celluloses, natural sponges, silica gels, glass, metals plastic, cellulose, cross-linked dextrans, such as those sold under the tradename Sephadex (Pharmacia) and agarose gel, such as gels sold under the tradename Sepharose (Pharmacia), which is a hydrogen bonded polysaccharide-type agarose gel, and other such resins and solid phase supports known to those of skill in the art.

The resins or other solid supports are modified such that they present a functional group to the safety-catch linker that is capable of forming a covalent bond with the Y group of the linker. Functional groups which possess such capability include, but are not limited to, carboxylic acids, haloalkyl, preferably chloromethyl groups, halosilanes, and amino, hydroxyl or thiol groups. Thus, for linkers where Y is NH₂, OH or SH, solid supports which possess carboxylic acid, chloromethyl or halosilane functionality are preferred, forming amide, ester, thioester, aminomethyl, ether, thioether, aminosilane, silylether or silylthioether linkages. Linkers where Y is COOH react preferentially with solid supports presenting amino, hydroxyl or thiol groups, forming amide, ester or thioester linkages. Preferred solid supports include polystyrene/divinylbenzene resins, such as Wang resin, which is 4-(hydroxymethyl)-phenoxymethylcopoly-(styrene-1 % divinylbenzene (DVB)) resin, chlorotrityl (2-chlorotritylchloride copolystyrene-DVB) resin, aminomethyl (copolystyrene-DVB) resin and Merrifield (chloromethylated copolystyrene-DVB) resin. A more preferred solid support is aminomethylated polystyrene.

### B. Preparation of Safety-Catch Linkers

The safety-catch linkers provided herein may be prepared by the methods described below or other methods apparent to those of skill in the art. Any similar safety-catch linkers may be synthesized by minor modification of the methods or by choosing appropriate starting materials.

A suitable X² precursor, such as homophthalic anhydride, maleic anhydride or 3,3-dimethylglutaric anhydride, is reduced to the corresponding diol with, e.g., lithium aluminum hydride. Mono-protection of this diol with, e.g., tert-butyldimethylsilyl chloride, provides a monoalcohol. Oxidation of this alcohol to the corresponding aldehyde may be accomplished by treatment with, in the case of allylic and benzylic alcohols, manganese dioxide. In the case of other alcohols, the aldehyde may be synthesized by oxidation under Swern conditions (see, e.g., Omura *et al.* Tetrahedron **1978**, 34, 1651). Reductive amination of this aldehyde with an X¹ precursor (e.g., an ω-aminoacid ester, ω-amino-α-alcohol, α,ω-diamine or ω-amino-α-thiol), such as methoxymethyl 6-aminohexanoate (prepared from N-Boc-6-aminohexanoic acid by esterification with methoxy methyl chloride and removal of the Boc group with aqueous acid), in the presence of a reducing agent, such as sodium cyanoborohydride, sodium borohydride, or hydrogen over palladium on carbon, affords a secondary amine which has protected X¹ and X² precursor groups as its substituents. At the terminus of the X¹ precursor group is an acid-labile ester (e.g., a methoxymethyl ester), while at the terminus of the X² precursor group is a protected alcohol (e.g., a tertbutydimethylsilyl ether).

This amine is then protected with an amine protecting group, such as, but not limited to, carbamates, amines, sulfonamides, sulfenamides and silyl derivatives. For example, the amine may be derivatized as the corresponding N-hydroxy-(R³R⁴)aminocarbamate by reaction with, e.g., N-piperidinyl 4-nitrophenyl carbonate (Pipoc-OPNP) (see, e.g., Handford, *et al.* J. Chem. Soc. **1965**, 6814-6827; Stevenson *et al.* J. Chem. Soc. C **1969**, 2389-2398). Removal of the alcohol protecting group, e.g., the tert-butyldimethylsilyl ether, under standard conditions known to those of skill in the art, e.g., treatment with tetrabutylammonium fluoride, gives a primary alcohol that is oxidized to the corresponding acid with, e.g., pyridinium dichromate in N,N-dimethylformamide. This acid is esterified as its R³ ester by reaction with an alcohol (R³OH, e.g., 9-fluorenylmethanol), N,N'-dicyclohexylcarbodiimide and 4-dimethylaminopyridine. Conversion of the ester on the X¹ chain (e.g., the methoxymethyl ester) to the corresponding carboxylic acid by hydrolysis under acidic (e.g., HCOOH) conditions completes the synthesis of the safety-catch linker.

### C. Methods for use of Safety-Catch Linkers

### 1. Linking Substrates to Resins and other solid supports

The safety-catch linkers are designed for linkage of substrates to be modified, such as, but not limited to, alcohols, amines and thiols, to solid supports, which are intended for use in any methods in which it is desired to link the substrates to a support, and particularly methods in which linkage is desired only temporarily. In particular, linkage of such compounds and moieties is desired in solid phase chemical and biological protocols, such as solid phase syntheses, particularly solid phase syntheses of small organic molecules.

Linkage of the substrate to the resin or other solid support is preferably effected by formation of a covalent bond between the substrate (i.e., alcohol, amine or thiol) and the acyl moiety (i.e., the carboxylic acid, ester, thioester, thiaester, dithioester, imino ether or imino thioether) of the safety-catch linker. Formation of the covalent bond may be achieved by any method known to those of skill in the art. For example, the acyl group may be converted to an acyl chloride, which is then reacted with the amine, alcohol or thiol. Alternatively, covalent bond formation may be achieved under dehydrative conditions, e.g., in the presence of a carbodiimide such as dicyclohexylcarbodiimide or diisopropylcarbodiimide.

### 2. Resins

The resins include any solid supports for solid phase synthesis that are known to those of skill in the art. It is understood that the resins and other solid supports contemplated are those that are suitable for use as a support for retaining molecules during syntheses or reactions. See, e.g., U.S. Patent No. 4,908,405; U.S. Patent No. 5,292,814; Butz et al. (1994) Peptide Res. 7:20-23; Kleine et al. (1994) Immunobiol. 190:53-66; Piskin et al, (1994), Chapter 18 "Nondegradable and Biodegradable Polymeric Particles" in Diagnostic Biosensor Polymers, ACS Symp.Series 556, Usmani et al. Eds, American Chemical Society, Washington, DC; Bayer et al, (1994) in Pept.: Chem., Struct. Biol., Proc. Am. Pept. Symp., 13th, Hodges, et al. eds., pp.156-158; Zhang et al. (1993) Pept. 1992. Proc. Eur. Pept. Symp., 22nd, Schneider, et al., eds. pp. 432-433; llg et al. (1994) Macromolecules, pp. 2778-83; Zeppezauer et al. (1993) Z. Naturforsch.. B: Chem. Sci. 48:1801-1806; Rapp et al. (1992) Pept. Chem. 1992, Proc. Jpn. Symp., 2nd, Yanaihara, ed., pp. 7-10; Nokihara et al. (1993) Shimadzu Hyoron 50:25-31; Wright et al. (1993) Tetrahedron Lett. 34:3373-3376; Bayer et al. (1992) Poly(Ethylene Glycol) Chem. Harris, ed., pp. 325-45; Rapp et al. (1990) Innovation Perspect. Solid Phase Synth. Collect. Pap., Int. Symp., 1st, Epton, ed., pp. 205-10; Rapp et al. (1992) Pept.: Chem. Biol.. Proc. Am. Pept. Symp., 12th. Smith et al., eds., pp. 529-530; Rapp et al. (1989) Pept., Proc. Eur. Pept. Symp., 20th, Jung et al., eds., pp. 199-201; Bayer et al. (1986) Chem. Pept. Proteins 3: 3-8; and Bayer et al. (1983) Pept.: Struct. Funct.. Proc. Am. Pept. Symp., 8th, Hruby et al. eds., pp. 87-90.

The resins or other solid supports are any that are suitable for solid phase or combinatorial chemical synthesis, such as, but not limited to, inorganics, natural polymers, and synthetic polymers, including, but not limited to: cellulose, cellulose derivatives, acrylic resins, glass that is derivatized to render it suitable for use a support, silica gels, polystyrene, gelatin, polyvinyl pyrrolidone, co-polymers of vinyl and acrylamide, polystyrene cross-linked with divinylbenzene or the like (see, Merrifield Biochemistry **1964**, 3, 1385-1390), polyacrylamides, latex gels, polystyrene, dextran, rubber, silicon, plastics, nitrocellulose, celluloses, natural sponges, silica gels, glass, metals plastic, cellulose, cross-linked dextrans, such as those sold under the tradename Sephadex (Pharmacia) and agarose gel, such as gels sold under the tradename Sepharose (Pharmacia), which is a hydrogen bonded polysaccharide-type agarose gel, and other such resins and solid phase supports known to those of skill in the art.

The resins or other solid supports are modified such that they present a functional group to the safety-catch linker that is capable of forming a covalent bond with the Y group of the linker. Functional groups which possess such capability include, but are not limited to, carboxylic acids, haloalkyl, preferably chloromethyl groups, halosilanes, and amino, hydroxyl or thiol groups. Thus, for linkers where Y is NH₂, OH or SH, solid supports which possess carboxylic acid, chloromethyl or halosilane functionality are preferred, forming amide, ester, thioester, aminomethyl, ether, thioether, aminosilane, silylether or silylthioether linkages. Linkers where Y is COOH react preferentially with solid supports presenting amino, hydroxyl or thiol groups, forming amide, ester or thioester linkages. Preferred solid supports include polystyrene/divinylbenzene resins (available from Fluka Chemical Corp., Milwaukee, WI), such as Wang resin, which is 4-(hydroxymethyl)phenoxymethylcopoly-(styrene-1 % divinylbenzene (DVB)) resin, chlorotrityl (2-chlorotritylchloride copolystyrene-DVB) resin, aminomethyl (copolystyrene-DVB) resin and Merrifield (chloromethylated copolystyrene-DVB) resin. A more preferred solid support is aminomethylated polystyrene.

### 3. Substrates

Suitable substrates for use in the methods include any compounds that are capable of forming a covalent bond with an acyl derivative, such as a carboxylic acid, ester, thioester, thiaester, dithioester, imino ether or imino thioether. Preferred substrates include alcohols, amines and thiols. Substrates for which the methods provided herein are particularly useful include, but are not limited to, compounds that are unstable toward the conditions required for cleavage of the substrate, following modification, from known linkers. Such substrates are subject to degradation, rearrangement or other transformation during cleavage from known linkers, making solid phase synthesis or modification of these substrates difficult or impossible. Particularly preferred substrates are compounds such as natural products, e.g., TAXOL, or other complex organic compounds.

### 4. Use of the solid supports

The methods of use of the safety-catch linkers provided herein in solid phase and combinatorial solid phase synthesis are provided. The methods herein may be included as part of synthetic protocols for synthesizing libraries of compounds. Production of libraries of compounds can be achieved by using the methods to produce a plurality of compounds in accord with any desired combinatorial synthesis protocol. Such protocols can involve the use of multiple resins or other solid supports, multiple reagents and modification of the substrate in diverse ways using various methods and reagents. The resins or other solid supports may be pins, beads, or any solid support known to the skilled artisan. The supports may be used in conjunction with multi-well plates, where a different combination of reagents and modification of methods is used in each well to provide libraries of interest (see, e.g., International PCT application Nos. WO 97/35198 and WO 94/11388). In an exemplary embodiment, a single compound is prepared in each well and the compounds formed in the wells collectively form the libraries. Alternatively, following each transformation, the supports bearing the resin-bound products are pooled and randomly split into a number of groups. Each group is then modified using a different method or reagent. This pool/split/modify protocol is then repeated a desired number of times to afford the diverse libraries of interest.

It is contemplated herein that the above protocols may be used in conjunction with microreactors, such as microvessels that are hollow, preferably tubular, devices fabricated from a polymeric material, such as PFTE, EFTE, or other such material, and treated to render the outer surface suitable for linking substrates using the safety-catch linkers provided herein. These microvessels include the MICROTUBE® microreactors and MICROKAN® microreactors which contain particular matrix materials that are suitably derivatized for linking the compounds (see, co-pending U.S. Patent Application Serial Nos. 08/711,426, 08/709,435, 08/723,423, 08/633,410, 08/669,252, 08/726,703, 08/743,984, 08/741,685, 08/857,800, 08/826,253, 08/912,998, 08/958,254 and 08/881,248 and International Patent Application Publication Nos. WO 97/49653, WO 96/36436 and WO 97/12680).

These devices and other containers may include a bar code, particularly a two-dimensional optical bar code in place of or in addition to an electromagnetic tag. For instance, a bar code may be imprinted on the outer surface of the MICROTUBE® microreactor or on the base of each well of a microplate or elsewhere.

It is further contemplated that the methods, in a combinatorial approach, include the use of chemical, radioactive, electromagnetic, optical, or other means for tagging each support to assist in deconvolution of the produced libraries. Such tagging assists in identification of active compounds following high throughput screening of the libraries of compounds generated.

The methods involve linking of a substrate, such as an amine, thiol or alcohol, to a solid support, such as a resin, using the safety-catch linkers provided herein and, following modification of the substrate, cleaving the modified substrate from the safety-catch linker by a cyclization protocol involving the two step process of (a) activation of the linker through removal of R¹; and (b) cleavage through cyclization induced by treatment with a mild base, such as an amine, or an alkali or alkaline earth metal phosphate, hydrogen phosphate, dihydrogen phosphate, hydroxide, carbonate or hydrogen carbonate.

In one embodiment, the methods involve: linking a safety-catch linker to a resin or other solid support through the Y group of the linker; removing R² from the resin-bound safety-catch linker; linking a substrate, such as, but not limited to, an alcohol, amine or thiol, to the resulting carboxyl group of the resin-bound linker; and, following modification of the substrate, removing the modified substrate from the resin using a two-step process of (a) activating the safety-catch linker toward cleavage by removal of R¹, and (b) cleaving the modified substrate from the activated safety-catch linker by cyclization induced by treatment with a base, such as, but not limited to, an amine, or an alkali or alkaline earth metal phosphate, hydrogen phosphate, dihydrogen phosphate, hydroxide, carbonate or hydrogen carbonate.

In this embodiment, removal of R¹ is performed by any method known to those of skill in the art and will depend upon the nature and structure of R¹ (see, generally, Greene, "Protective Groups in Organic Synthesis" (John Wiley & Sons, Inc., New York, NY)). In preferred embodiments, the safety-catch linkers provided herein are of formula III. Thus, in preferred embodiments, R¹ is R³R⁴NOC(O)- and may be removed under mild reducing conditions, such as, but not limited to, treatment with a transition metal or a transition metal salt in aqueous acid, sodium dithionite in aqueous acid, hydrogenation over a transition metal catalyst or electrolysis. A more preferred method for removal of R¹ is treatment with sodium dithionite in aqueous formic acid (see Examples for details).

The following examples are included for illustrative purposes only and are not intended to limit the scope of the invention.

### EXAMPLE 1

### Preparation of safety-catch linker N-[2-(9-fluorenylmethoxycarbonylmethyl)benzyl]-N-Pipoc-6-aminohexanoic acid

**A.2-Hydroxymethylphenethyl alcohol**
   Homophthalic anhydride (24.80 g, 85% pure, 130.0 mmol, 1 equiv.) was dissolved in dry THF (200 mL). LiAIH₄ (1.0 M in THF, 184 mL, 184 mmol, 1.4 equiv.) was added dropwise at room temperature. The resulting brown solution was stirred at room temperature for 1 hour and 75 °C overnight. The reaction was cooled to room temperature with a cold water bath and carefully quenched with methanol (25 mL). The quenched reaction solution was then slowly poured into a vigorously stirred 10% NaOH aqueous solution (500 mL) and stirred for 30 min. (until the solution became clear). The mixture was extracted with diethyl ether (200 mL x 3). The extracts were combined, dried over magnesium sulfate, concentrated, and flash column chromatographed on silica gel with 40-80% EtOAc / petroleum ether to yield a pale oil (13.43 g, 68%): R_{*f*} = 0.55, EtOAc; ¹H NMR (500 MHz, CDCl₃) δ 7.34 (d, *J* = 7.6 Hz, 1 H), 7.30 (d, *J* = 6.6 Hz, 1 H), 7.20-7.25 (m, 2 H), 4.68 (s, 2 H), 3.93 (t, *J* = 5.7 Hz, 2 H), 2.98 (t, *J* = 5.7 Hz, 2 H).
**B. 2-Hydroxymethylphenethyl *t*-butyldimethylsilyl ether**
   The diol prepared in Example 1.A. (13.00 g, 85.42 mmol, 1.0 equiv.) was dissolved in dry dichloromethane (50 mL). Triethylamine (14.29 mL, 102.4 mmol, 1.2 equiv.) and 4-dimethylaminopyridine (1.04 g, 8.51 mmol, 0.1 equiv.) were added. A solution of tert-butyldimethylsilyl chloride (12.88 g, 85.45 mmol, 1.0 equiv.) in dichloromethane (170 mL) was slowly added to the stirred reaction at room temperature over an 8 h period. The reaction was stirred for overnight, diluted with ether (400 mL), washed with saturated sodium bicarbonate (100 mL x 3) and brine (100 mL x 1), dried over magnesium sulfate, concentrated, and flash chromatographed on silica gel with 5-15% EtOAc / petroleum ether to yield 6.34 g (28%) of the desired silyl ether, and its regio-isomer and starting material, which were recycled. A yield of 14.40 g (64%) of silyl ether was obtained with only 3 cycles: R*f* = 0.60, 10% EtOAc / petroleum ether; ¹H NMR (500 MHz, CDCl₃) δ 7.19-7.35 (m, 4 H), 4.64 (s, 2 H), 3.90 (t, *J* = 5.8 Hz, 2 H), 2.95 (t, *J* = 5.8 Hz, 2 H), 0.79 (s, 9 H), -0.06(s, 6 H).
**C. 2-Formylphenethyl *t*-butyldimethylsilyl ether**
   The benzyl alcohol prepared in Example 1.B. (14.40 g, 54.04 mmol, 1.0 equiv.) was dissolved in hexane and treated with activated MnO₂ (55.30 g, 85% pure, 541 mmol, 10 equiv.) at room temperature for 14 h. The solid was filtered off and the colorless solution was concentrated and briefly chromatographed on silica gel with 0-5% EtOAc / hexane to yield 12.37 g (87%) of the desired aldehyde as a colorless oil: R*f* = 0.55, 40% EtOAc / petroleum ether; ¹H NMR (500 MHz, CDCl₃) δ 10.29 (s, 1 H), 7.82-7.86 (m, 1 H), 7.25-7.54 (m, 3 H), 3.85 (t, *J* = 6.6 Hz, 2 H), 3.24 (t, *J* = 6.6 Hz, 2 H), 0.82 (s, 9 H), -0.06(s, 6 H).
**D. Methoxymethyl *N*-(2-*t*-butyldimethylsiloxyethylbenzyl)-6-aminohexanoate**
   The benzyl aldehyde prepared in Example 1.C. (1.25 g, 4.73 mmol, 1.0 equiv.) and methoxymethyl 6-aminohexanoate (1.00 g, 5.68 mmol, 1.2 equiv.) were dissolved in anhydrous trimethylorthoformate (20 mL) and stirred at room temperature for 20 h. Sodium cyanoborohydride (0.60 g, 9.55 mmol, 2.0 equiv.) was dissolved in anhydrous trimethylorthoformate (10 mL) and added dropwise into the reaction at room temperature. After 10 min of stirring, acetic acid (0.30 mL, 1% v/v) was added dropwise and the reaction was stirred at room temperature for 7 h. The reaction solution was diluted with EtOAc (200 mL), washed with saturated sodium bicarbonate (50 mL x 3) and brine (50 mL x 1), dried over magnesium sulfate, concentrated, and chromatographed on silica gel with 1-5% MeOH / DCM to yield 0.91 g (46%) of the desired product as an oil: R*f* = 0.33, 5% MeOH / DCM; ¹H NMR (500 MHz, CDCl₃) δ 7.32-7.10 (m, 4 H), 5.22 (s, 2 H), 3.85 (s, 2 H), 3.82 (t, *J* = 7.0 Hz, 2 H), 3.46 (s, 3 H), 2.89 (t, *J* = 7.0 Hz, 2 H), 2.72 (t, *J* = 7.4 Hz, 2 H), 2.37 (t, *J* = 3.7 Hz, 2 H), 1.79-1.30 (m, 6 H), 0.84 (s, 9 H), -0.03(s, 6 H); LRMS (electrospray) *m/z* [M + H] ⁺ 424, calcd for C₂₃H₄₂NO₄Si 424.
**E. Methoxymethyl *N*-(2-t-butyldimethylsiloxyethylbenzyl)-*N*-Pipoc-6-aminohexanoate**
   The secondary amine prepared in Example 1.D. (0.90 g, 2.12 mmol, 1.0 equiv.) was dissolved in anhydrous DMF (20 mL). Diisopropylethylamine (0.56 mL, 3.20 mmol, 1.5 equiv.) and *N*-piperidinyl 4-nitrophenyl carbonate (Pipoc-OPNP) (0.79 g, 3.00 mmol, 1.4 equiv.) were added sequentially. The reaction was stirred at room temperature overnight, diluted with diethyl ether (200 mL), washed with saturated sodium bicarbonate (50 mL x 3) and brine (50 mL x 1), dried over magnesium sulfate, concentrated, and chromatographed on silica gel with 20-40% EtOAc / petroleum ether to yield the desired product as an oil (0.56 g, 48%): R*f* = 0.25, 40% EtOAc / petroleum ether; ¹H NMR (500 MHz, CDCl₃) δ 7.30-7.10 (m, 4 H), 5.22 (s, 2 H), 4.51 (br s, 2 H), 3.77 (t, *J* = 6.8 Hz, 2 H), 3.46 (s, 3 H), 3.40 (br s, 2 H), 3.18 (br s, 2 H), 2.84 (t, *J* = 6.8 Hz, 2 H), 2.60 (br s, 2 H), 2.32 (t, *J* = 7.5 Hz, 2 H), 1.85-1.50 (m, 12 H), 0.86 (s, 9 H), -0.03(s, 6 H); LRMS (electrospray) *m/z* [M + H] ⁺ 551, calcd for C₂₉H₅₁N₂O₆Si 551.
**F. Methoxymethyl *N*-(2-hydroxyethylbenzyl)-*N*-Pipoc-6-aminohexanoate**
   Tetrabutylammonium fluoride (2.0 mL, 1.0 M in THF, 2.00 mmol, 2.0 equiv.) was added to a THF (5 mL) solution of the TBS ether prepared in Example 1.E. (0.56 g, 1.02 mmol, 1.0 equiv.) at room temperature. After stirring at room temperature for 1 h, the reaction was diluted with water (100 mL), extracted with diethyl ether (50 mL x 3). The extracts were combined, dried over magnesium sulfate, concentrated, and chromatographed on silica gel with 50-100% EtOAc / petroleum ether to yield the desired alcohol as an oil (0.17 g, 38%): R*f* = 0.40, EtOAc; ¹H NMR (500 MHz, CDCl₃) δ 7.35-7.13 (m, 4 H), 5.22 (s, 2 H), 4.55 (br s, 2 H), 3.81 (br t, *J* = 6.4 Hz, 2 H), 3.46 (s, 3 H), 3.40 (br s, 2H), 3.16 (br s, 2 H), 2.91 (t, *J* = 6.4 Hz, 2 H), 2.62 (br s, 2 H), 2.33 (t, *J* = 7.5 Hz, 2 H), 1.82-1.20 (m, 12 H); LRMS (electrospray) *m/z* [M + H] ⁺ 437, calcd for C₂₃H₃₇N₂O₆ 437.
**G. Methoxymethyl *N*-[2-19-fluorenylmethoxycarbonylmethyl)benzyl]-*N*-Pipoc-6-aminohexanoate**
   The phenethyl alcohol prepared in Example 1.F. (0.17 g, 0.39 mmol, 1.0 equiv.) was treated with pyridinium dichromate (0.88 g, 2.34 mmol, 6.0 equiv.) in DMF (5 mL) at room temperature for 48 h. The solution was diluted with saturated NaH₂PO₄ (pH 3-4, 100 mL) and extracted with EtOAc (50 mL x 4). The extracts were combined, dried over magnesium sulfate, and concentrated under vacuum. The dried crude product was then dissolved in dry dichloromethane (2 mL) and treated with excess of 9-fluorenylmethanol, 4-dimethylaminopyridine, and N,N'-dicyclohexylcarbodiimide at room temperature for 10 h. The solution was concentrated and the residue was chromatographed on a preparative silica gel TLC with 50% EtOAc / petroleum ether to yield 18 mg (8% overall) of the desired ester as an oil: R*f* = 0.75, EtOAc; ¹H NMR (500 MHz, CDCl₃) δ 7.85-7.25 (m, 12 H), 5.20 (s, 2 H), 4.52 (br s, 2 H), 4.36 (br d, *J* = 6.9 Hz, 2 H), 4.15 (t, *J* = 6.9 Hz, 1 H), 3.76 (br s, 2 H), 3.44 (s, 3 H), 3.50-2.50 (m br s, 6 H), 2.28 (t, *J* = 7.5 Hz, 2 H), 2.00-1.20 (m, 12 H); LRMS (electrospray) *m/z* [M + H]⁺ 629, calcd for C₃₇H₄₅N₂O₇ 629.
**H. *N*-[2-(9-fluorenylmethoxycarbonylmethyl)benzyl]-*N*-Pipoc-6-aminohexanoic acid**
   The MOM ester prepared in Example 1.G. (18 mg, 0.029 mmol, 1 equiv.) was treated with formic acid at room temperature for 6 h. The solution was concentrated under vacuum to yield 17 mg (quantitative) of the desired carboxylic acid: R*f* = 0.40, 10% MeOH / DCM; LRMS (electrospray) *m/z* [M + H] ⁺ 585, calcd for C₃₅H₄₁N₂O₆ 585.

### EXAMPLE 2

### N-[2-(9-fluorenylmethoxycarbonylmethyl)benzyl]-N-Pipoc-6-aminohexanoyl-NH-polystyrene resin

The safety-catch linker prepared in Example 1 (17 mg, 0.029 mmol, 1.0 equiv.) was reacted with excess aminomethyl polystyrene resin (0.40 g, 0.45 mmol/g, 0.18 mmol, 6.0 equiv.), diisopropylethylamine (0.02 mL, 0.12 mmol, 4.0 equiv.), and PyBop (30 mg, 0.058 mmol, 2.0 equiv.) in dry dichloromethane (4 mL) at room temperature for 20 h. TLC analysis of the supernatant indicated that all the linker had been consumed. The resin was washed with THF and diethyl ether alternately (10 mL x 4) and dried under vacuum. The excess amino groups on the resin were then capped with Ac₂O / diisopropylethylamine (0.5 M each, 4 mL) at room temperature for 0.5 h. Kaiser test showed negative. The capped resin was washed and dried as above to yield the desired resin.

### EXAMPLE 3

### General activation procedure for safety-catch linkers

The resin prepared in Example 2 was activated by treatment with Na₂S₂O₄ in THF/H₂O/(HOAc or HCOOH) at room temperature for a period of time, washed with (HOAc or HCOOH)/THF three times, and dried under vacuum. The amount of 9-fluorenylmethanol (FmOH) released in the activation and washings was measured by HPLC analysis. The conditions for activation and washing were optimized to minimize release of FmOH in these steps. This allows for complete removal of the Pipoc protecting group by-products and the activation reagents prior to cleavage of the desired compound (in this example FmOH) from the linker. Optimal conditions that were found were activation with 0.1 M Na₂S₂O₄ in THF/H₂O/HCOOH at room temperature for 1 h, followed by washing with 10% HCOOH/THF three times. See the following table for details.

### EXAMPLE 4

### General cleavage procedure for safety-catch linkers

The activated resin prepared in Example 3 was treated with 0.5 M Et₃N/benzene at room temperature for 1 h. The resin was filtered off and the filtrate was analyzed for released 9-fluorenylmethanol by HPLC analysis. Control experiments showed that, in the absence of activation, no FmOH was released from the resin under these cleavage conditions. Residual FmOH (FmOH still attached to the linker following cleavage under these conditions) was determined by treatment of the recovered resin with 20% piperidine/DMF followed by measurement of the released 9-methylidenefluorene by HPLC. See the following table for details.

| | activation reagents | activation time (h) | washing conditions | % FmOH in activation | % FmOH in washing | % FmOH in cleavage | residual FmOH (%) |
|---|---|---|---|---|---|---|---|
| 1 | 0.25 M Na₂S₂O₄ HOAc:H₂O (3:1) | 15 | 10% HOAc | 0 | 0 | 27 | 26 |
| 2 | 0.25 M Na₂S₂O₄ HOAc:H₂O (3:1) | 64 | 10% HOAc | 0 | 0 | 30 | 3 |
| 3 | 0.1 M Na₂S₂O₄ THF:H₂O:HOAc (5:5:1) | 14 | 10% HOAc | 72 | 1.2 | 22 | N.D. |
| 4 | 0.1 M Na₂S₂O₄ THF:H₂O:HOAc (5:5:1) | 1 | 10% HOAc | 14 | 1.2 | 71 | N.D. |
| 5 | 0.1 M Na₂S₂O₄ THF:H₂O:HOAc (6:6:5) | 1 | 10% HOAc | 0 | 4 | 62 | 27 |
| 6 | 0.1 M Na₂S₂O₄ THF:H₂O:HOAc (6:6:5) | 2 | 30% HOAc | 4.6 | 1.2 | 64 | 3.5 |
| 7 | 0.1 M Na₂S₂O₄ THF:H₂O:HCOO H (6:6:5) | 2 | 10% HCOOH | 0 | 0 | 83 | 3.5 |
| 8^{a} | none | 0 | none | 0 | 0 | 0 | 100 |

| | | | | | | | |
|---|---|---|---|---|---|---|---|
| ^{a} Resin without activation was treated with 0.5 M Et₃N/benzene at room temperature for up to 24 hours. | | | | | | | |

Since modifications will be apparent to those of skill in this art, it is intended that this invention be limited only by the scope of the appended claims.

## Claims

1. A safety-catch linker, comprising X², wherein
X² is a bivalent group wherein the atoms at either end of X² are held in proximity to one another such that formation of a covalent bond between the atoms at either end of X² is facilitated.

2. The safety-catch linker of claim 1 that has the formula : 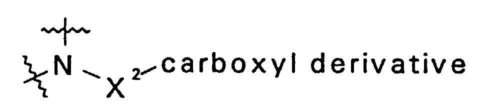 wherein:
X² is chosen from (cis)-alkenylene, aralkenylene, alkylenearalkylene and an alkylene group that is substituted with two substituents at the same atom of the alkylene chain; and
the carboxyl acid derivative is chosen from esters, thioesters, thiaesters, dithioesters, imino ethers and iminothioethers.

3. The safety-catch linker of claim 1 or 2, wherein X² is 1,1-, 2,2-, or 3,3-(R¹⁰R¹¹)propylene, 1,1-, 2,2-, 3,3- or 4,4-(R¹⁰R¹¹)butylene, (cis)-1-butenylene, (cis)-2-butenylene, (cis)-3-butenylene, (cis)-1-propenylene, (cis)-2-propenylene, 1-methylene-1,2-phenylene, 1-ethylene-1,2-phenylene or 1,2-di(methylene)-1,2-phenylene; wherein R¹⁰ and R¹¹ are each independently alkyl, or together form alkylene or alkylidene.

4. The safety-catch linker of claim 3, wherein X² is 1,2-di(methylene-1,2-phenylene.

5. The safety-catch linker of claim 1 that has formula I: 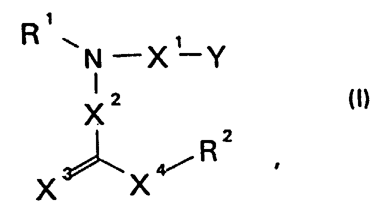 wherein :
R¹ is hydrogen or an amine protecting group;
R² is hydrogen or a carboxyl protecting group;
Y is chosen from COOH, NH₂, OH and SH;
X¹ and X² are each independently chosen from alkylene, alkenylene, alkynylene, arylene, heteroarylene, aralkylene, heteroalkylene, cycloalkylene, heterocyclylene, alkylenearalkylene, alkyleneheteroaralkylene, alkylenecycloalkylene, alkyleneheterocyclylene, alkylenecycloalkylenealkylene or alkyleneheterocyclyenealkylene, and are unsubstituted or substituted with one or more substituents designated Z;
each Z is independently chosen from halogen, hydroxy, nitrile, nitro, formyl, mercapto, carboxy, alkyl, haloalkyl, polyhaloalkyl, aminoalkyl, diaminoalkyl, alkenyl having 1 or 2 double bonds, alkynyl having 1 or 2 triple bonds, cycloalkyl, cycloalkylalkyl, aryl, heteroaryl, arylalkyl, heteroarylalkyl, alkylidene, arylalkylidene, alkylcarbonyl, arycarbonyl, heteroarylcarbonyl, alkoxycarbonyl, alkoxycarbonylalkyl, aryloxycarbonyl, aryloxycarbonylalkyl, aminocarbonyl, alkylaminocarbonyl, dialkylaminocarbonyl, arylaminocarbonyl, diarylaminocarbonyl, arylalkylaminocarbonyl, alkoxy, aryloxy, perfluoroalkoxy, alkenyloxy, alkynyloxy, arylalkoxy, amino, aminoalkyl, alkylaminoalkyl, dialkylaminoalkyl, arylaminoalkyl, diarylamino-alkyl, alkylamino, dialkylamino, arylamino, diarylamino, alkylarylamino, alkylcarbonylamino, alkoxycarbonylamino, arylcarbonylamino, aryloxycarbonylamino, azido, alkylthio, arylthio, perfluoroalkylthio, thiocyano, isothiocyano, alkylsulfinyl, alkylsulfonyl, arylsulfinyl, arylsulfonyl, aminosulfonyl, alkylaminosulfonyl, dialkylaminosulfonyl, arylaminosulfonyl and diarylaminosulfonyl, or any two independently selected Z substituents together form alkylene, alkenylene or alkynylene;
X³ is chosen from oxy, thio and NR²⁰, wherein R²⁰ is hydrogen, alkyl, aryl, heteroaryl, cycloalkyl or heterocyclyl; and
X⁴ is oxy or thio.

6. The safety-catch linker of claim 5, wherein :
R¹ is chosen from hydrogen, alkoxycarbonyl, aryloxycarbonyl, arylalkoxycarbonyl, heteroaryloxycarbonyl, cycloalkoxycarbonyl, heterocyclyloxycarbonyl, heteroarylalkoxycarbonyl, cycloalkylalkoxycarbonyl, heterocyclylalkoxycarbonyl, arylalkyl, diarylalkyl, triarylalkyl, alkylcarbonyl, arylcarbonyl, aryalkylcarbonyl, heteroarylcarbonyl, heteroarylalkylcarbonyl, R³R⁴NOC(O)- and R³S(O)ₙ-; R³ and R⁴ are each independently chosen from alkyl, alkenyl, alkynyl, aryl, arylalkyl, heteroaryl and heteroarylalkyl, or together form alkylene or alkenylene; and n is from 0 to 2;
R² is chosen from hydrogen, alkyl, alkenyl, alkynyl, aryl, heteroaryl, aralkyl, heteroaralkyl, cycloalkyl, heterocyclyl, trialkysilyl, aryldialkylsilyl, diarylalkysilyl and triarylsilyl; and
R¹ and R² are unsubstituted with one or more independently selected Z substituents.

7. The safety-catch linker of claim 5 or 6, wherein :
X² is chosen from alkylene, alkenylene, aralkylene, heteroaralkylene, alkylenearalkylene, alkyleneheteroaralkylene, alkylenecycloalkylene, alkyleneheterocyclylene, alkylenecycloalkylenealkylene and alkyleneheterocyclylenealkylene, and is unsubstituted or substituted with one or more independently selected Z substituents.

8. The safety-catch linker of claim 7, wherein :
X² is 1,1-, 2,2-, or 3,3-(R¹⁰R¹¹)propylene or 1,1-, 2,2-, 3,3-, or 4,4-(R¹⁰R¹¹)butylene, where R¹⁰ and R¹¹ are each independently alkyl, or together form alkylene or alkylidene.

9. The safety-catch linker of claim 8, wherein :
R¹⁰ and R¹¹ are methyl or R¹⁰ and R¹¹ together form pentylene, butylene, propylene or ethylene.

10. The safety-catch linker of claim 7, wherein :
X² is chosen from (cis)-1-butenylene, (cis)-2-butenylene, (cis)-3-butenylene, (cis)-1-propenylene and (cis)-2-propylene.

11. The safety-catch linker of claim 7, wherein :
X² is chosen from 1-methylene-1,2-phenylene, 1-ethylene-1,2-phenylene and 1,2-di(methylene)-1,2-phenylene.

12. The safety-catch linker of claim 5, wherein X³ and X⁴ are both oxy.

13. The safety-catch linker of claim 12, wherein :
R¹ is chosen from hydrogen, alkoxycarbonyl, aryloxycarbonyl, arylalkoxycarbonyl, heteroaryloxycarbonyl, cycloalkoxycarbonyl, heterocyclyloxycarbonyl, heteroarylalkoxycarbonyl, cycloalkylalkoxycarbonyl, heterocyclylalkoxycarbonyl, arylalkyl, diarylalkyl, triarylalkyl, alkylcarbonyl, arylcarbonyl, arylalkycarbonyl, heteroarylcarbonyl, heteroarylalkylcarbonyl, R³R⁴NOC(O)- and R³S(O)ₙ-; R³ and R⁴ are each independently chosen from alkyl, alkenyl, alkynyl, aryl, arylalkyl, heteroaryl and heteroaryalkyl, or together form alkylene or alkenylene; and n is from 0 to 2;
R² is chosen from hydrogen, alkyl, alkenyl, alkynyl, aryl, heteroaryl, aralkyl, heteroaralkyl, cycloalkyl, heterocyclyl, trialkylsilyl, aryldialkylsilyl, diarylalkylsilyl and triarylsilyl; and
R¹ and R² are unsubstituted or substituted with one or more independently selected Z substituents.

14. The safety-catch linker of claim 13, wherein :
X² is chosen from alkylene, alkenylene, aralkylene, heteroaralkylene, alkylenearalkylene, alkyleneheteroaralkylene, alkylenecycloalkylene, alkyleneheterocyclylene, alkylenecycloalkylenealkylene and alkyleneheterocyclylenealkylene, and is unsubstituted or substituted with one or more independently selected Z substituents.

15. The safety-catch linker of claim 14, wherein :
X² is 1,1-, 2,2-, or 3,3-(R¹⁰R¹¹)propylene or 1,1-, 2,2-, 3,3- or 4,4-(R¹⁰R¹¹)butylene, where R¹⁰ and R¹¹ are each independently alkyl, or together form alkylene or alkylidene.

16. The safety-catch linker of claim 15, wherein :
R¹⁰ and R¹¹ are methyl or R¹⁰ and R¹¹ together form pentylene, butylene, propylene or ethylene.

17. The safety-catch linker of claim 14, wherein :
X² is chosen from (cis)-1-butenylene, (cis)-2-butenylene, (cis)-3-butenylene, (cis)-1-propenylene and (cis)-2-propenylene.

18. The safety-catch linker of claim 14, wherein :
X² is chosen from 1-methylene-1,2-phenylene, 1-ethylene-1,2-phenylene and 1,2-di(methylene)-1,2-phenylene.

19. The safety-catch linker of claim 18, wherein :
X² is 1,2-di(methylene)-1,2-phenylene.

20. The safety-catch linker of any of claim 16, 17 or 19, wherein :
R¹ is chosen from hydrogen, alkoxycarbonyl, aryloxycarbonyl, aralkoxycarbonyl, hetercyclyloxycarbonyl, heterocyclylalkoxycarbonyl and R³R⁴NOC(O)-;
R³ and R⁴ are each independently chosen from alkyl, aryl, aralkyl, heteroaryl and heteroaralkyl, or together form alkylene or alkenylene;
R² is chosen from hydrogen, alkyl, alkenyl, alkynyl, aryl, heteroaryl, aralkyl, heteroaralkyl, cycloalkyl and heterocyclyl; and
X¹ is chosen from alkylene, alkenylene, alkenylene, alkynylene, arylene, heteroarylene, aralkylene and heteroaralkylene.

21. The safety-catch linker of claim 20, wherein :
R¹ is R³R⁴NOC(O)-;
R³ and R⁴ are each independently chosen from alkyl, or together from alkylene;
R² is hydrogen, alkyl or aralkyl; and
X¹ is chosen from alkylene and arylene.

22. The safety-catch linker of claim 21, wherein :
R³ and R⁴ together form pentylene;
R² is 9-fluorenylmethyl; and
X¹ is pentylene.

23. The safety-catch linker of claim 22, wherein Y is COOH.

24. The safety-catch linker according to any preceding claim that is covalently bonded to a solid support.

25. The safety-catch linker of claim 24, wherein the solid support is an aminomethylated polystyrene.

26. A method for solid phase synthesis, comprising :
(a) linking a substrate to a solid support via a safety-catch linker according to any preceding claim; and
(b) modifying the substrate and cleaving the modified substrate from the safety-catch linker under mild conditions.

27. The method of claim 26, wherein the safety-catch linker is as claimed in any one of claims 5 to 23.

28. The method of claim 27, wherein modifying the substrate and cleaving the modified substrate from the safety-catch linker under mild conditions comprises the steps of:
(i) activating of the linker through removal of R¹; and
(ii) cleaving the substrate from the linker by treatment with a mild base; wherein :
the substrate is an amine, alcohol or thiol;
the solid support is chosen from supports used for solid phase or combinatorial chemical synthesis and is modified such that it presents a functional group to the safety-catch linker that forms a covalent bond with the linker; and
the mild base is an amine, or is an alkali metal or an alkaline earth metal phosphate, hydrogen phosphate, dihydrogen phosphate, hydroxide, carbonate or hydrogen carbonate.

29. The method of any of claims 26 to 28, wherein :
the solid support is aminomethylated polystyrene; and
the mild base is triethylamine.

30. A solid support, comprising the safety-catch linker of any of claims 1 to 23.
